(19) Europäisches Patentamt
European Patent Office
Office européen des brevets

(11) Numéro de publication: **0 199 660 B1**

(12) # FASCICULE DE BREVET EUROPEEN

(45) Date de publication du fascicule du brevet: **08.02.89**

(51) Int. Cl.[4]: **C 07 C 43/225**, C 07 C 149/34, C 07 C 41/22

(21) Numéro de dépôt: **86420092.8**

(22) Date de dépôt: **27.03.86**

(54) **Procédé de préparation de dérivés aromatiques 1,1 difluoro perhalogénoalcoxylés ou 1,1 difluoro perhalogénothioalkylés.**

(30) Priorité: **29.03.85 FR 8504756**

(43) Date de publication de la demande: **29.10.86 Bulletin 86/44**

(45) Mention de la délivrance du brevet: **08.02.89 Bulletin 89/6**

(84) Etats contractants désignés: **AT BE CH DE FR GB IT LI LU NL SE**

(56) Documents cités:

**EP-A- 0 124 002**
**EP-A- 0 168 344**
**US-A- 2 803 665**
**US-A- 3 207 792**

**JOURNAL OF ORGANIC CHEMISTRY, vol. 44, no. 16, 3 août 1979, pages 2907-2910, American Chemical Society, US; A.E. FEIRING: "Chemistry in hydrogen fluoride". 7. A novel synthesis of aryl trifluoromethyl ethers"**
**JOURNAL OF THE AMERICAN CHEMICAL SOCIETY, vol. 65, no. 11, 13 novembre 1943, pages 2043-2066; J.H. SIMONS et al.: "Fluorine derivatives of acetophenone and ethylbenzene"**

(73) Titulaire: **RHONE-POULENC CHIMIE, 25, quai Paul Doumer, F-92408 Courbevoie Cédex (FR)**

(72) Inventeur: **Desbois, Michel, 526, rue du Bois, F-69140 Rillieux la Pape (FR)**

(74) Mandataire: **Le Pennec, Magali et al, RHONE-POULENC INTERSERVICES Service Brevets Chimie 25, Quai Paul Doumer, F-92408 Courbevoie Cédex (FR)**

EP 0 199 660 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de dérivés aromatique 1,1 difluoro perhalogénoalcoxylés ou 1,1 difluoro perhalogénothioalkylés par fluoration de dérivés aromatiques perhalogénoalcoxylés ou perhalogénothioalkylés dont la chaîne alkyle contient au moins deux atomes de carbone.

On connaît dans l'art antérieur des documents décrivant la préparation de composés voisins mais dont la chaîne alkylée est attachée directement au cycle par une liaison covalente. C'est ainsi que Simons et Therman décrivent dans le «Journal of American Society 65, 2064-6 (1943)» la synthèse du tétrafluorochlorophényléthane par fluoration du 1,1,1 trifluoro-2,2 dichlorophényléthane dans l'acide fluorhydrique liquide en présence de $SbCl_5$.

Mac Bee décrit dans le brevet US N° 2 639 299 la préparation de décafluorodiéthylbenzène par fluoration du décachlorodiéthylbenzène dans l'acide fluorhydrique liquide en présence de $ScCl_5$.

Les rendements des réactions de fluoration sont, dans les deux documents préalablement cités, de l'ordre de 15% ce qui exclut toute possibilité d'application industrielle.

Le brevet US 2 803 665 décrit un procédé de fluoration de composés perhalogénoalcoxylés, essentiellement aliphatiques, mais néanmoins pouvant comporter des groupes benzyliques, par un système catalytique choisi parmi $SbF_3$, HF ou le couple HF/tri-halogénure d'antimoine. L'acide de Lewis est utilisé en quantité légèrement inférieure à la molarité du composé à fluorer mais peu différente.

Le catalyseur est complètement transformé et non récupérable en fin de réaction.

Le journal of Organic Chemistry, vol. 44, n° 16, pages 2907-2910 décrit la préparation de trfluorométhoxybenzènes par action conjuguée de $CCl_4$ et de HF en présence éventuellement d'un catalyseur de Lewis type $BF_3$ ou $SbX_5$ qui a cependant tendance à former des goudrons. Ce procédé décrit toujours la méthylation du groupe OH d'un phénol, puis l'échange Cl → F.

Le brevet US 3 207 792 décrit la fluoration-échange de trichlorométhylthiobenzène ($\emptyset SCCl_3 \rightarrow \emptyset SCF_3$) par HF seul, $SbF_3$ seul, ou le mélange des deux.

La fluoration-échange ($\emptyset ACCl_3 \rightarrow \emptyset ACF_3$) des trichlorométhoxybenzènes ou des trichlorométhylthiobenzènes peut aussi être effectué par HF seul, $SbX_5$ seul, ou les deux.

La présente concommittante des deux catalyseurs n'a pas d'effet synergique sur le type de réaction catalysée.

Dans le cas de la présente invention ce n'est pas le cas, la fluoration-échange du groupe $-CX_2-$ situé en α de l'hétéroatome et en α d'un groupe trihalogénométhyle ($\emptyset ACX_2-CX_3$) n'est pas possible avec HF seul ni avec $SbX_5$ seul. La présence des deux composés est absolument nécessaire et ceci n'était pas évident à la lecture des documents cités.

Le brevet européen n° 124 002 dans son exemple 9 décrit la réaction d'échange de chlorofluorobenzodioxane avec le couple HF et $SbCl_5$.

Le rendement en produit perfluoré est d'environ 20%, les produits secondaires sont des dérivés résineux qui ne sont pas recyclables. Cet essai qui est un essai comparatif prouve l'effet surprenant de la fluoration avec un acide de Lewis seul qui est l'objet dudit brevet par rapport à la même fluoration réalisée avec un double système catalytique composé d'un acide de Lewis et d'acide fluorhydrique.

Cet essai comparatif prouve que les dérivés benzyldioxane ne sont pas comparables aux dérivés de la présente invention. En effet, le cycle dioxane est très fragile, il n'est pas stable dans l'acide fluorhydrique et a tendance à donner des résines. Les composés de la présente invention sont par contre très stables et ne peuvent être fluorés que par le couple Sb $Cl_5$ et HF et non par Sb $Cl_5$ seul.

Il est encore connu d'après le brevet européen EP 169 344 publié postérieurement au dépôt de la présente demande de procéder à la fluoration-échange des α, α dichloroalkylphényléthers par une quantité catalytique d'un dérivé de l'antimoine dans une solution d'acide fluorhydrique. La présente invention a fait l'objet d'un disclaimer afin de s'écarter de cette demande pour les pays désignés conjointement avec le brevet européen EP 168 344.

Elle a trait en effet à un procédé de préparation de dérivés aromatiques 1,1 difluoro perhalogénoalcoxylés ou 1,1 difluoro perhalogénothioalkylés caractérisé en ce qu'on met en contact un dérivé perhalogénoalcoxylé ou perhalogénothioalkylé dont la chaîne alkyle contient au moins deux atomes de carbone avec un acide de Lewis dans de l'acide fluorhydrique liquide anhydre.

On entend au sens de la présente invention par dérivés perhalogénoalcoxylés ou perhalogénothioalkylés, d'une part ces produits proprement dits et, d'autre part, leurs homologues correspondant à la présence d'un ou plusieurs radicaux substituant le noyau aromatique.

Les dérivés perhaloghénoalcoxylés ou perhalogénothioalkylés concernés par la présente invention ont pour formule générale:

$$(R)_p\text{-Ar-}[ACX_1X_2\text{-}(CX_3X_4)_mX_5]_n \quad (I)$$

dans laquelle:

- chaque X identique ou différent représente Cl, Br, ou F l'un au moins des atomes $X_1$ ou $X_2$ n'étant pas du fluor,
- A représente l'oxygène ou le soufre,
- m représente un nombre entier supérieur ou égal à 1 est inférieur ou égal à 3,
- n est un nombre entier égal ou supérieur à 1 et inférieur ou égal à 2,
- R représente au moins un élément choisi parmi le groupe comprenant l'hydrogène, les halogènes, les radicaux alkyle, alcoxy, nitro, carbonyle, carboxyle, thioalkyle ayant de 1 à 6 atomes de carbone, halogénoalkyle, halogénoalcoxy, halogénothioalkyle, phényle ou phénoxy éventuellement substitué,
- p est un nombre entier supérieur ou égal à 1 et inférieur ou égal à 3.

Les composés de formule I dans laquelle n = 1, m = 1, p = 1 ou 2 sont de préférence mis en œuvre

dans la présente invention. Ceux par lesquels n = 1, p = 2, $X_3 = X_4 = X_5 = F$, Ar représente un groupe phényle et R représente le chlore sont encore plus particulièrement concernés.

Les acides de Lewis sont choisis parmi $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_5$, $WF_6$. On préfère utiliser le pentachlorure d'antimoine.

Le disclaimer pour les pays suivants: AT, DE, I, GB, LI, FR, NL, BE consiste en ce que lorsque R représente l'hydrogène, les halogènes, le groupe nitro, l'acide de Lewis ne représente pas $SbCl_5$ ni $SbF_5$.

Le procédé selon l'invention est mis en œuvre de préférence utilisant une quantité d'acide fluorhydrique telle que le rapport molaire de l'acide fluorhydrique au composé de formule I est compris entre 5 et 50. Plus particulièrement, ce rapport est compris entre 10 et 30.

On préfère utiliser une quantité de pentachlorure d'antimoine telle que le rapport molaire pentachlorure d'antimoine au dérivé perhalogénoalcoxylés ou perhalogénothioalkylés soit compris entre 1 et 20% et de préférence entre 5 et 15%.

Une quantité supéreure n'est pas exclue de l'invention mais n'apporte pas d'avantages particuliers.

Le procédé selon l'invention est de préférence mis en œuvre à une température comprise entre 0 et 180°C et de préférence entre 80 et 150°C.

La température sera adaptée par l'homme de l'art aux catalyseurs et aux composés aromatiques de départ.

Les dérivés aromatiques 1,1 difluoro perhalogénoalcoxylés ou 1,1 difluoro perhalogénothioalkylés obtenus selon le procédé de l'invention ont pour formule générale (II)

$$(R')_p\, Ar[ACF_2(CX_3X_4)_mX_5]_n \qquad (II)$$

ans laquelle:

- Ar, A, m, n ont la signification précédente,
- $R'_1$ a la signification précédente quand $R_1$ représente l'hydrogène, les radicaux alkyle, alcoxy, thioalkyle, phénole, phénoxy, carbonyle ou carboxyle,
- Lorsque R représente un radical halogénoalkyle, halogénoalcoxy, halogénothioalkyle, il est transformé en radical partiellement ou totalement fluoré.

On peut citer comme exemple de produits de formule (II):

Le pentafluoroéthoxybenzène, le pentafluoroéthylthiobenzène,
le p-chloropentafluoroéthoxybenzène,
le dichloro-2,4 pentafluoroéthoxybenzène,
le p-fluoropentafluoroéthoxybenzène,
le p-fluoropentafluoroéthylthiobenzène,
le m-trifluorométhylpentafluoroéthoxybenzène,
le p-nitropentafluoroéthylthiobenzène,
le m-chloropentafluoroéthylthiobenzène,
le bis(pentafluoroéthoxy)-2,4 benzène.
le bis(pentafluoroéthylthio)-2,4 benzène
l'heptafluoroisopropyloxybenzène,
l'1,1-difluoro-2,2,2 trifluoroéthoxybenzène.

Les dérivés aromatiques 1,1 difluoro perhalogénoalcoxylés ou 1,1 difluoro perhalogénothioalkylés sont utilisés comme intermédiaire de synthèse dans l'industrie pharmaceutique vétérinaire ou phytosanitaire et dans l'industrie des lubrifiants US 4 366 168, EP 44 808, US 3 265 741.

La présente invention sera plus complètement décrite à l'aide des exemples suivants qui ne doivent pas être considérés comme limitatifs de l'invention.

*Exemple 1: p-chloro(pentafluoroéthoxy)benzène*

Dans un réacteur inox de 250 ml agité par barreau aimanté, on introduit successivement, sous atmosphère inerte ($N_2$):

- 3 g (0,01 mole) de $SbCl_5$ anhydre
- 28 g (0,1 mole) de p-chloro (1,1-dichloro 2,2,2-trifluoroéthoxy)benzène
- 25 g (1,25 mole) de HF anhydre.

Le mélange réactionnel est porté, sous agitation, à 120°C pendant 3 h 15 mn. La pression s'élève jusqu'à 26 bars. Après réaction, le réacteur est réfroidi vers 0°C, décomprimé (on laisse échapper l'acide chlorhydrique). Le mélange réactionnel obtenu est coulé sur 85 g de glace pilée. Après extraction par trois fois 100 ml de $CH_2Cl_2$, lavage des phases organiques à l'eau permuttée (100 ml) et filtration, on mesure par titrage infra-rouge environ 24 g de p-chloro(pentafluoroéthoxy)benzène, soit un rendement analytique d'environ 97,5%. Par chromatographie en phase gazeuse, on note l'absence du composé de départ.

Après évaporation du solvant, on récupère 17,2 g de parachloro(pentafluoroéthoxy)benzène (rendement: 70%).

*Exemple 1 comparatif*

On utilise le même mode opératoire qu'à l'exemple 1 mais en supprimant $SbCl_5$.

Après 3 heures de réaction, on récupère intégralement le produit de départ et on n'obtient aucune trace de p. chloro(pentafluoroéthoxy)benzene.

*Exemple 2: p-nitro(pentafluoroéthylthio)bentène*

On opère de manière identique à l'exemple 1 avec les conditions et composés suivants:

| | |
|---|---|
| – p-nitro(1,1-dichloro--2,2,2 trifluoroéthylthio)benzène: | 5 g - 0,017 mole |
| – HF anhydre: | 10 g - 0,5 mole |
| – $SbCl_5$: | 0,8 g - 0,0027 mole |
| – Température: | 100°C |
| – Durée: | 5 heures |

Les analyses par chromatographie phase gazeuse (normalisation à 100) et spectrométrie de masse montrent la présence de 50% de p-nitro(pentafluoroéthylthio)benzène.

*Exemple 3: (Pentafluoroéthoxy)benzène*

On opère de manière identique à l'exemple 1 avec les conditions et composés suivants:

- 1,1-dichloro-2,2,2 trifluoroéthoxybenzène: 10 g - 0,04 mole
- HF anhydre: 20 g - 1 mole
- $SbCl_5$: 0,6 g - 0,002 mole
- Température: 130°C
- Durée: 4 heures

Les analyses par chromatographie phase gazeuse (normalisation à 100) et spectrométrie de masse montrent la présence de 76% de (pentafluoroéthoxy)benzène.

*Exemple 4: m-chloro(pentafluoroéthylthio)benzène*

On opère de manière identique à l'exemple 1 avec les conditions et composés suivants:

- m-chloro(1,1-dichloro-2,2,2 trifluoroéthylthio)benzène: 3 g - 0,01 mole
- HF anhydre: 10 g - 0,5 mole
- $SbCl_5$: 0,3 g - 0,001 mole
- Température: 150°C
- Durée: 6 heures

Les analyses par chromatographie phase gazeuse (normalisation à 100) et spectrométrie de masse montrent la présence de 22% de m-chloro(pentafluoroéthylthio)benzène.

*Exemple 5: p-chloro(pentafluoroéthoxy)benzène*

On opère de manière identique à l'exemple 1 avec les conditions et composés suivants:

- p-chloro(1,1-dichloro-2,2,2 trifluoroéthoxybenzène: 42 g - 0,15 mole
- HF anhydre: 50 g - 2,5 moles
- $BF_3$: 10 bars à 20°C
- Température: 100°C
- Durée: 3 h 45

Les analyses par chromatographie phase gazeuse (normalisation à 100) et spectrométrie de masse montrent la présence de 39,5% de p-chloro(pentafluoroéthoxy)benzène.

*Exemple 6: p-chloro(pentafluoroéthoxy)benzène*

On opère de manière identique à l'exemple 1 avec les conditions et composés suivants:

- p-chloro(1,1-dichloro-2,2,2 trifluoroéthoxy)benzène: 42 g - 0,15 mole
- HF anhydre: 50 g - 2,5 moles
- $SnCl_4$: 5,8 g - 0,022 mole
- Température: 150°C
- Durée: 6 heures

Les analyses par chromatographie phase gazeuse (normalisation à 100) et spectrométrie de masse montrent la présence de 38% de p-chloro(pentafluoroéthoxy)benzène.

*Exemple 7: p-chloro(pentafluoroéthoxy)benzène*

On opère de manière identique à l'exemple 1 avec les conditions et composés suivants:

- p-chloro(1,1-dichloro-2,2,2 trifluoroéthoxy)benzène: 10,5 g - 0,04 mole
- HF anhydre: 40 g - 2 moles
- $TiCl_4$: 1 g - 0,005 mole
- Température: 150°C
- Durée: 22 heures

Les analyses par chromatographie phase gazeuse (normalisation à 100) et spectrométrie de masse montrent la présence de 18% de p-chloro(pentafluoroéthoxy)benzène.

*Exemple 8: 1,1-difluoro-(2,2,2 trichloroéthoxy)-benzène*

On opère de manière identique à l'exemple 1 avec les conditions et composés suivants:

- Pentachloroéthoxybenzène: 44,1 g - 0,15 mole
- HF anhydre: 50 g - 2,5 moles
- $SbCl_5$: 6,7 g - 0,0225 mole
- Température: 120°C
- Durée: 3 heures

Les analyses par chromatographie phase gazeuse (normalisation à 100) et spectrométrie de masse montrent la présence de 79% de 1,1-difluoro-(2,2,2 trichloroéthoxy)benzène.

**Revendications pour les Etats Contractants:**
AT, DE, IT, GB, CH, LI, FR, NL, BE.

1. Procédé de préparation de dérivés aromatiques 1,1 difluoroalcoxylés ou 1,1 difluorothioalkylés, caractérisé en ce qu'on met en présence un dérivé aromatique perhalogénoalcoxylé ou perhalogénothioalkylé répondant à la formule générale (I)

$$R_p\text{-}Ar\text{-}[A\text{-}CX_1X_2\text{-}(CX_3X_4)_mX_5]_n$$

dans laquelle:

- A représente l'oxygène ou le soufre
- Ar représente un radical aromatique mono ou polycyclique
- Chaque X représente un halogène choisi parmi Cl, Br, F, un au moins de $X_1$ ou $X_2$ n'étant pas F
- n est un nombre entier égal ou supérieur à 1 et inférieur ou égal à 2
- m est un nombre entier égal ou supérieur à 1 et inférieur ou égal à 3
- R représente au moins un élément choisi parmi le groupe comprenant l'hydrogène, les halogènes, les radicaux alkyle, alcoxy, thioakyle, ayant de 1 à 6 atomes de carbone, halogénoalkyle, halogénoalcoxy, halogénothioalkyle, phényle ou phenoxy éventuellement substitué, nitro, carbonyle, carboxyle
- p est un nombre entier égal ou supérieur à 1 et inférieur ou égal à 3,

avec une quantité catalytique d'un acide de Lewis choisi parmi $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_6$, $WF_6$ dans l'acide fluorhydrique liquide anhydre et caractérisé en ce

que lorsque R représente l'hydrogène, les halogènes, le groupe nitro, l'acide de Lewis ne représente pas $SbCl_5$ ni $SbF_5$.

2. Procédé selon la revendication 1, caractérisé en ce que dans la formule I:

– $X_1$, $X_2$ représentent chacun le chlore
– n est égal à 1
– m est égal à 1
– p est égal à 1 ou 2
– R représente l'hydrogène ou un halogène
– Ar représente un cycle phényle
– $X_3$, $X_4$, $X_5$ représent chacun le fluor.

3. Procédé selon la revendication 2, caractérisé en ce que la formule I représente le parachloro(1,1--dichloro-2,2,2 trifluoroéthoxy)benzène.

4. Procédé selon la revendication 1, caractérisé en ce que l'acide de Lewis est du pentachlorure d'antimoine.

5. Procédé selon la revendication 1, caractérisé en ce que le rapport molaire du pentachlorure d'antimoine au dérivé perhalogénoalcoxylé ou perhalogénothioalkylé est compris entre 1 et 20% et de préférence entre 5 et 15%.

6. Procédé selon la revendication 1, caractérisé en ce que le rapport molaire de l'acide fluorhydrique au dérivé perhalogénoalcoxylé ou perhalogénothioalkylé est compris entre 5 et 50 et de préférence entre 10 et 30.

7. Procédé selon la revendication 1, caractérisé en ce que la température de la réaction est comprise entre 0 et 180°C et de préférence entre 80 et 150°C.

**Revendications pour les Etats Contractants:** SE, LU

1. Procédé de préparation de dérivés aromatiques 1,1 difluoroalcoxylés ou 1,1 difluorothioalkylés, caractérisé en ce qu'on met en présence un dérivé aromatique perhalogénoalcoxylé ou perhalogénothioalkylé répondant à la formule générale (I)

$$R_p\text{-}Ar\text{-}[A\text{-}CX_1X_2\text{-}(CX_3X_4)_mX_5]_n$$

dans laquelle:

– A représente l'oxygène ou le soufre
– Ar représente un radical aromatique mono ou polycyclique
– Chaque X représente un halogène choisi parmi Cl, Br, F, un au moins de $X_1$ ou $X_2$ n'étant pas F
– n est un nombre entier égal ou supérieur à 1 et inférieur ou égal à 2
– m est un nombre entier égal ou supérieur à 1 et inférieur ou égal à 3
– R représente au moins un élément choisi parmi le groupe comprenant l'hydrogène, les halogènes, les radicaux alkyle, alcoxy, thioalkyle, ayant de 1 à 6 atomes de carbone, halogénoalkyle, halogénoalcoxy, halogénothioalkyle, phényle ou phénoxy éventuellement substitué, nitro, carbonyle, carboxyle
– p est un nombre entier égal ou supérieur à 1 et inférieur ou égal à 3,

avec une quantité catalytique d'un acide de Lewis choisi parmi $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_6$, $WF_6$ dans l'acide fluorhydrique liquide anhydre.

2. Procédé selon la revendication 1, caractérisé en ce que dans la formule I:

– $X_1$, $X_2$ représentent chacun le chlore
– n est égal à 1
– m est égal à 1
– p est égal à 1 ou 2
– R représente l'hydrogène ou un halogène
– Ar représente un cycle phényle
– $X_3$, $X_4$, $X_5$ représent chacun le fluor.

3. Procédé selon la revendication 2, caractérisé en ce que la formule I représente le parachloro(1,1--dichloro-2,2,2 trifluoroéthoxy)benzène.

4. Procédé selon la revendication 1, caractérisé en ce que l'acide de Lewis est du pentachlorure d'antimoine.

5. Procédé selon la revendication 1, caractérisé en ce que le rapport molaire du pentachlorure d'antimoine au dérivé perhalogénoalcoxylé ou perhalogénothioalkylé est compris entre 1 et 20% et de préférence entre 5 et 15%.

6. Procédé selon la revendication 1, caractérisé en ce que le rapport molaire de l'acide fluorhydrique au dérivé perhalogénoalcoxylé ou perhalogénothioalkylé est compris entre 5 et 50 et de préférence entre 10 et 30.

7. Procédé selon la revendication 1, caractérisé en ce que la température de la réaction est comprise entre 0 et 180°C et de préférence entre 80 et 150°C.

**Patentansprüche für die Vertragsstaaten:**
AT, BE, CH, DE, FR, GB, IT, LI, NL

1. Verfahren zur Herstellung 1,1-difluoralkoxylierter oder 1,1-difluorthioalkylierter aromatischer Verbindungen, dadurch gekennzeichnet, dass man eine aromatische perhalogenalkoxylierte oder perhalogenthioalkylierte Verbindung gemäss der allgemeinen Formel (I)

$$R_p\text{-}Ar\text{-}[A\text{-}CX_1X_2\text{-}(CX_3X_4)_mX_5]_n$$

in der:

– A Sauerstoff oder Schwefel darstellt
– Ar einen aromatischen mono- oder polycyclischen Rest darstellt
– X jeweils ein Halogen darstellt ausgewählt aus Cl, Br und F, wobei wenigstens eines aus $X_1$ oder $X_2$ nicht F ist
– n eine ganze Zahl gleich oder grösser als 1 und kleiner oder gleich 2 ist
– m eine ganze Zahl gleich oder grösser als 1 und kleiner oder gleich 3 ist,
– R wenigstens ein Element darstellt ausgewählt aus der Gruppe umfassend Wasserstoff, die Halogene, die Reste Alkyl, Alkoxy, Thioalkyl, mit 1 bis 6 Kohlenstoffatomen, Halogenalkyl, Halogenalkoxy, Halogenthioalkyl, Phenyl oder Phenoxy, gegebenenfalls substituiert, Nitro, Carbonyl, Carboxyl,

– p eine ganze Zahl gleich oder grösser als 1 und kleiner oder gleich 3 ist,

mit einer katalytischen Menge einer Lewis-Säure ausgewählt aus $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_6$, $WF_6$ in flüssiger wasserfreier Fluorwasserstoffsäure umsetzt, und dadurch gekennzeichnet, dass wenn R darstellt Wasserstoff, die Halogene oder die Nitrogruppe, die Lewis-Säure nicht $SbCl_5$ oder $SbF_5$ ist.

2. Verfahren nach Anspruch 1, dadurch gekennzeichnet, dass in der Formel I:

– $X_1$ und $X_2$ jeweils Chlor darstellen
– n gleich 1 ist
– m gleich 1 ist
– p gleich 1 oder 2 ist
– R Wasserstoff oder ein Halogen darstellt
– Ar einen Phenylring darstellt
– $X_3$, $X_4$ und $X_5$ jeweils Fluor darstellen.

3. Verfahren nach Anspruch 2, dadurch gekennzeichnet, dass die Formel I Parachlor(1,1-Dichlor-2,2,2-trifluorethoxy)benzol darstellt.

4. Verfahren nach Anspruch 1, dadurch gekennzeichnet, dass die Lewis-Säure Antimonpentachlorid ist.

5. Verfahren nach Anspruch 1, dadurch gekennzeichnet, dass das molare Verhältnis von Antimonpentachlorid zur perhalogenalkoxylierten oder perhalogenthioalkylierten Verbindung zwischen 1 und 20% und vorzugsweise zwischen 5 und 15% beträgt.

6. Verfahren nach Anspruch 1, dadurch gekennzeichnet, dass das molare Verhältnis von Fluorwasserstoffsäure zur perhalogenalkoxylierten oder perhalogenthioalkylierten Verbindung zwischen 5 und 50 und vorzugsweise zwischen 10 und 30 beträgt.

7. Verfahren nach Anspruch 1, dadurch gekennzeichnet, dass die Reaktionstemperatur zwischen 0 und 180°C und vorzugsweise zwischen 80 und 150°C liegt.

**Patentansprüche für die Vertragsstaaten:** SE, LU

1. Verfahren zur Herstellung aromatischer 1,1-Difluoralkoxy- oder 1,1-Difluorthioalkylverbindungen, dadurch gekennzeichnet, dass man eine aromatische perhalogenalkoxylierte oder perhalogenthioalkylierte Verbindung entsprechend der allgemeinen Formel (I)

$$R_p\text{-}Ar\text{-}[A\text{-}CX_1X_2\text{-}(CX_3X_4)_mX_5]_n$$

in der:
– A Sauerstoff oder Schwefel darstellt
– Ar einen aromatischen mono- oder polycyclischen Rest darstellt
– X jeweils ein Halogen darstellt ausgewählt aus Cl, Br und F, wobei wenigstens $X_1$ oder $X_2$ nicht F ist
– n eine ganze Zahl gleich oder grösser als 1 und kleiner oder gleich 2 ist
– m eine ganze Zahl gleich oder grösser als 1 und kleiner oder gleich 3 ist,
– R wenigstens ein Element darstellt ausgewählt aus der Gruppe umfassend Wasserstoff, die Halogene, die Reste Alkyl, Alkoxy, Thioalkyl, mit 1 bis 6 Kohlenstoffatomen, Halogenalkyl, Halogenalkoxy, Halogenthioalkyl, Phenyl oder Phenoxy, gegebenenfalls substituiert, Nitro, Carbonyl, Carboxyl,
– p eine ganze Zahl gleich oder grösser als 1 und kleiner oder gleich 3 ist,

mit einer katalytischen Menge einer Lewis-Säure ausgewählt aus $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_6$, $WF_6$ in flüssiger wasserfreier Fluorwasserstoffsäure umsetzt.

2. Verfahren nach Anspruch 1, dadurch gekennzeichnet, dass in der Formel I:

– $X_1$ und $X_2$ jeweils Chlor darstellen
– n gleich 1 ist
– m gleich 1 ist
– p gleich 1 oder 2 ist
– R Wasserstoff oder ein Halogen darstellt
– Ar einen Phenylring darstellt
– $X_3$, $X_4$ und $X_5$ jeweils Fluor darstellen.

3. Verfahren nach Anspruch 2, dadurch gekennzeichnet, dass die Formel I Parachlor(1,1-Dichlor-2,2,2-trifluorethoxy)benzol darstellt.

4. Verfahren nach Anspruch 1, dadurch gekennzeichnet, dass die Lewis-Säure Antimonpentachlorid ist.

5. Verfahren nach Anspruch 1, dadurch gekennzeichnet, dass das molare Verhältnis von Antimonpentachlorid zur perhalogenalkoxylierten oder perhalogenthioalkylierten Verbindung zwischen 1 und 20% und vorzugsweise zwischen 5 und 15% beträgt.

6. Verfahren nach Anspruch 1, dadurch gekennzeichnet, dass das molare Verhältnis von Fluorwasserstoffsäure zur perhalogenalkoxylierten oder perhalogenthioalkylierten Verbindung zwischen 5 und 50 und vorzugsweise zwischen 10 und 30 beträgt.

7. Verfahren nach Anspruch 1, dadurch gekennzeichnet, dass die Reaktionstemperatur zwischen 0 und 180°C und vorzugsweise zwischen 80 und 150°C liegt.

**Claims for the Contracting States:**
AT, DE, IT, GB, CH, LI, FR, NL, BE

1. Process for the preparation of aromatic 1,1-difluoroalkoxy or 1,1-difluorothioalkyl derivatives, characterized in that an aromatic perhaloalkoxy or perhalothioalkyl derivative corresponding to the general formula (I)

$$R_p\text{-}Ar\text{-}[A\text{-}CX_1X_2\text{-}(CX_3X_4)_mX_5]_n$$

in which:

– A denotes oxygen or sulphur
– Ar denotes a monocyclic or polycyclic aromatic radical
– each X denotes a halogen chosen from Cl, Br and F, at least one of $X_1$ or $X_2$ being other than F
– n is an integer equal to or greater than 1 and equal to or smaller than 2

– m is an integer equal to or greater than 1 and smaller than or equal to 3

– R denotes at least one member chosen from the group comprising hydrogen, the halogens and the alkyl, alkoxy, thioalkyl containing from 1 to 6 carbon atoms, haloalkyl, haloalkoxy, halothioalkyl, optionally substituted phenyl or phenoxy, nitro, carbonyl and carboxyl radicals, and

– p is an integer equal to or greater than 1 and smaller than or equal to 3,

is brought into contact with a catalytic quantity of a Lewis acid chosen from $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_6$ and $WF_6$ in anhydrous liquid hydrofluoric acid and characterized in that, when R denotes hydrogen, the halogens or the nitro group, the lewis acid does not denote $SbCl_5$ or $SbF_5$.

2. Process according to Claim 1, characterized in that, in the formula I:

- $X_1$ and $X_2$ both denote chlorine
- n is equal to 1
- m is equal to 1
- p is equal to 1 or 2
- R denotes hydrogen or a halogen
- Ar denotes a phenyl ring
- $X_3$, $X_4$ and $R_5$ all denote fluorine.

3. Process according to Claim 2, characterized in that the formula I denotes para-chloro-(1,1-dichloro-2,2,2-trifluoroethoxy)benzene.

4. Process according to Claim 1, characterized in that the Lewis acid is antimony pentachloride.

5. Process according to Claim 1, characterized in that the molar ratio of antimony pentachloride to the perhaloalkoxy or perhalothioalkyl derivative is between 1 and 20% and preferably between 5 and 15%.

6. Process according to Claim 1, characterized in that the molar ratio of hydrofluoric acid to the perhaloalkoxy or perhalothioalkyl derivative is between 5 and 50 and preferably between 10 and 30.

7. Process according to Claim 1, characterized in that the reaction temperature is between 0 and 180 and preferably between 80 and 150°C.

**Claims for the Contracting States:** SE, LU

1. Process for the preparation of aromatic 1,1-difluoroalkoxy or 1,1-difluorothioalkyl derivatives, characterized in that an aromatic perhaloalkoxy or perhalothioalkyl derivative corresponding to the general formula (I)

$$R_p\text{-}Ar\text{-}[A\text{-}CX_1X_2\text{-}(CX_3X_4)_mX_5]_n$$

in which:

– A denotes oxygen or sulphur

– Ar denotes a monocyclic or polycyclic aromatic radical

– each X denotes a halogen chosen from Cl, Br and F, at least one of $X_1$ or $X_2$ being other than F

– n is an integer equal to or greater than 1 and equal to or smaller than 2

– m is an integer equal to or greater than 1 and smaller than or equal to 3

– R denotes at least one member chosen from the group comprising hydrogen, the halogens and the alkyl, alkoxy, thioalkyl containing from 1 to 6 carbon atoms, haloalkyl, haloalkoxy, halothioalkyl, optionally substituted phenyl or phenoxy, nitro, carbonyl and carboxyl radicals, and

– p is an integer equal to or greater than 1 and smaller than or equal to 3,

is brough into contact with a catalytic quantity of a Lewis acid chosen from $BF_3$, $SbCl_5$, $SbF_5$, $SnCl_4$, $SnF_4$, $TiCl_4$, $TiF_4$, $NbF_5$, $TaF_5$, $PF_5$, $AsF_5$, $MoF_6$ and $WF_6$ in anhydrous liquid hydrofluoric acid.

2. Process according to Claim 1, characterized in that, in the formula I:

- $X_1$ and $X_2$ both denote chlorine
- n is equal to 1
- m is equal to 1
- p is equal to 1 or 2
- R denotes hydrogen or a halogen
- Ar denotes a phenyl ring
- $X_3$, $X_4$ and $R_5$ all denote fluorine.

3. Process according to Claim 2, characterized in that the formula I denotes para-chloro-(1,1-dichloro-2,2,2-trifluoroethoxy)benzene.

4. Process according to Claim 1, characterized in that the Lewis acid is antimony pentachloride.

5. Process according to Claim 1, characterized in that the molar ratio of antimony pentachloride to the perhaloalkoxy or perhalothioalkyl derivative is between 1 and 20% and preferably between 5 and 15%.

6. Process according to Claim 1, characterized in that the molar ratio of hydrofluoric acid to the perhaloalkoxy or perhalothioalkyl derivative is between 5 and 50 and preferably between 10 and 30.

7. Process according to Claim 1, characterized in that the reaction temperature is between 0 and 180°C and preferably between 80 and 150°C.